# EUROPEAN PATENT APPLICATION

(11) **EP 2 781 594 A1**
(43) Date of publication of application: **24.09.2014**
(21) Application number: 12849690.8
(22) Date of filing: 20.11.2012
(51) Int. Cl.: C12N 5/00, C12M 3/00

(54) **CELL CULTURE SUBSTRATE, AND METHOD FOR MANUFACTURING SAME**

(30) Priority: 20.11.2011 JP 2011269434
(71) Applicant: Tokyo Women's Medical University, Tokyo 162-8666 (JP)
(72) Inventor: FUKUMORI, Kazuhiro, Tokyo 178-0064 (JP); AKIYAMA, Yoshikatsu, Tokyo 162-8666 (JP); YAMATO, Masayuki, Tokyo 162-8666 (JP); OKANO, Teruo, Tokyo 162-8666 (JP)
(74) Representative: Regimbeau
(86) International application number: PCT/JP2012/080044
(87) International publication number: WO 2013/073707

(57) **Abstract**

A cell culture substrate is used comprising a photopolymerization initiator immobilized on a surface of the cell culture substrate, and a linear polymer immobilized on a part or the entirety of the surface via the photopolymerization initiator, and wherein the photopolymerization initiator is thioxanthone. Thereby, advantageously, a single type or multiple types of cells are efficiently cultured on specific regions of the culture substrate, and efficiently detached only by changing temperature on the surface of the substrate.

## Description

### TECHNICAL FILED

The present invention relates to a cell culture substrate useful in the fields of biology, medicine and the like, and relates to a manufacturing method thereof.

### BACKGROUND ART

To date, technologies for animal cell culture have made remarkable progress, leading to research and development of animal cells in various fields. Intended uses of animal cells include commercialization of initially developed cells themselves and substances produced therefrom. In addition to these, the followings are being attempted: effective pharmaceutical agents are designed by analyzing cells and their surface proteins; patient's cells which have been proliferated *ex vivo* or enhanced in their functions are returned to that patient for therapeutic purposes. Currently, a technology for culturing animal cells is one of the fields which have gathered much attention from many researchers.

Meanwhile, many of animal cells including human cells are adhesion-dependent. That is, animal cells first need to be adhered to something when they are cultured *ex vivo.* In this context, many researchers have previously designed device surfaces which are more preferred for cells. However, all of the technologies relate to those used during cell culture. Adhesion-dependent cultured cells themselves produce adhesive proteins when they adhere to something. Accordingly, when detaching these cells, the adhesive proteins have to be destroyed usually by enzymatic treatment in the conventional technology. In doing so, various cell-surface proteins unique to the cells which are produced during cell culture are also destroyed at the same time. However, currently there is no means for solving this serious problem, and no particular effort has been made to find it. A solution to this cell recovery problem is strongly demanded to dramatically advance the research and development of animal cells.

In this context, Patent Literature 1 describes a novel cell culture method comprising: culturing cells on a cell culture substrate at a temperature not more than a maximum critical solution temperature or not less than a minimum critical solution temperature, the cell culture substrate having a device surface on which a polymer having a maximum or minimum critical solution temperature of 0 to 80C in water is immobilized; then changing the temperature to not less than the maximum critical solution temperature or not more than the minimum critical solution temperature to detach the cultured cells without enzymatic treatment. Further Patent Literature 2 describes a method comprising: culturing skin cells at a temperature not more than a maximum critical solution temperature or not less than a minimum critical solution temperature using the above temperature responsive cell culture device; then changing the temperature to not less than the maximum critical solution temperature or not more than the minimum critical solution temperature to detach the cultured skin cells without significant damage. Furthermore, Patent Literature 3 describes a method of repairing a surface protein on a cultured cell using the above temperature responsive cell culture device. Use of the temperature responsive cell culture device has led to various novel advancements as compared with the conventional culture technology.

However, the technologies described above involve an immobilization process of a chemically inert engineered plastic surface using high energy such as an electron beam. To do this, a large-scale instrument such as an electron beam irradiator is required. Therefore, the resulting substrate is inevitably expensive. This has been a problem.

To date, some technologies have been developed in order to solve the above problem. Representative examples include methods of immobilizing a synthesized polymer having a specific molecular structure on a substrate surface as described in Nonpatent Literatures 1 and 2. However, none of them have reached a technical level in which cells can be cultured as effective as in the conventional cell culture substrate and cells can be detached simply by changing temperature as effective as in the temperature responsive cell culture substrate prepared with an electron beam and further cultured cells can be detached as a cell sheet when they become confluent. Accordingly, their technical levels need to be improved. Further, complicated processes have been required to immobilize different polymers in a certain pattern on a substrate, and to immobilize different amounts of a polymer in a certain pattern using these technologies. To date, technologies for cell culture have been diversified and sophisticated. Accordingly, a technology for functionalizing a surface of a cell culture substrate with simple method has been strongly required.

### CITATION LIST

### Patent Literature

Patent Literature 1 Japanese Patent Laid-Open No. H02-211865
Patent Literature 2: Japanese Patent Laid-Open No. H05-192138
Patent Literature 3: Japanese Patent Laid-Open No. 2008-220354

### Nonpatent Literature

Nonpatent literature 1: Soft Matter, 5, 2937-2946 (2009)
Nonpatent literature 2: Interface, 4, 1151-1157 (2007)

### SUMMARY OF INVENTION

The present invention has been made in order to solve the above problem in the conventional technology. That is, an object of the present invention is to provide a novel cell culture substrate developed by an approach completely different from the conventional technology. Further, another object of the present invention is to provide a manufacturing method thereof.

The present inventors have conducted research and development using various approaches in order to solve the above problem. As a result, surprisingly, the present inventors have found that a photopolymerization initiator can be easily immobilized on a surface of a cell culture substrate under a specific condition, and then a linear polymer can be immobilized via the initiator simply by irradiating the surface with light after a monomer solution is applied thereon. Further, the present inventors have found that this method can allow a linear polymer to be immobilized only on an irradiated region when a region on a substrate surface irradiated with light is changed, and also can allow different polymers or different amounts of a polymer to be easily immobilized in a certain pattern on the substrate surface. According to the present invention, the following may be achieved: for example, a complex pattern or shape pre-registered in a personal computer (hereinafter may be referred to as PC) may be projected with a projector so that the complex pattern or shape of different polymers or different amounts of a polymer can be easily formed on a substrate surface. The present invention has been completed based on the above findings.

That is, the present invention is defined as follows.

Item 1. A cell culture substrate, comprising a photopolymerization initiator immobilized on a surface of the cell culture substrate, and a linear polymer immobilized on a part or the entirety of the surface via the photopolymerization initiator, and wherein the photopolymerization initiator is thioxanthone.

Item 2. The cell culture substrate according to item 1, wherein the linear polymer immobilized on the surface of the substrate comprises at least one temperature responsive polymer having hydration force changing between 0 and 80°C.

Item 3. The cell culture substrate according to any one of items 1 to 2, wherein said at least one temperature responsive polymer is poly (*N-*isopropylacrylamide).

Item 4. The cell culture substrate according to any one of items 1 to 3, having a surface comprising two regions:
a region A in which the temperature responsive polymer is immobilized, and a region B comprising any one of (1) to (4) or any combination of two or three of (1) to (4):
   (1) a region in which a polymer having higher affinity to cells than that in the region A is immobilized,
   (2) a region in which a polymer having lower affinity to cells than that in the region A is immobilized,
   (3) a region in which the temperature responsive polymer is immobilized in an amount different from that in the region A,
   (4) a region in which a polymer responsive to a temperature different from that in the region A is immobilized.

Item 5. The cell culture substrate according to item 4, wherein the immobilized amount of the temperature responsive polymer on the surface of the region A is 0.8 to 10.0 µg/cm².

Item 6. The cell culture substrate according to any one of items 4, 5, wherein the region A and the region B on the surface of the substrate form a pattern.

Item 7. The cell culture substrate according to item 6, wherein the pattern comprises a line and space.

Item 8. A method of manufacturing a cell culture substrate, the method comprising: immobilizing a photopolymerization initiator on a surface of the cell culture substrate, irradiating the photopolymerization initiator-immobilized surface of the substrate with light having an emission wavelength peak of 400 nm or longer than 400 nm to immobilize a linear polymer on a part or the entirety of the surface at the initiator, wherein the photopolymerization initiator is thioxanthone.

Item 9. The method of manufacturing a cell culture substrate according to item 8, wherein different kinds of linear polymers and/or different amounts of linear polymers are immobilized on the surface of the substrate by changing light irradiation regions on the surface of the cell culture substrate without placing a shield on the surface of the cell culture substrate.

Item 10. The method of manufacturing a cell culture substrate according to any one of items 8 to 10, wherein the linear polymer immobilized on the surface of the substrate comprises at least one temperature responsive polymer having hydration force changing between 0 and 80°C.

Item 11. The method of manufacturing a cell culture substrate according to item 10, wherein said at least one temperature responsive polymers is poly(N-isopropylacrylamide).

Item 12. The method of manufacturing the cell culture substrate according to any one of items 8 to 11, wherein said cell culture substrate has a surface comprising two regions:
a region A in which the temperature responsive polymer is immobilized, and a region B comprising any one of (1) to (4) or any combination of two or three of (1) to (4):
   (1) a region in which a polymer having higher affinity to cells than that in the region A is immobilized,
   (2) a region in which a polymer having lower affinity to cells than that in the region A is immobilized,
   (3) a region in which the temperature responsive polymer is immobilized in an amount different from that in the region A,
   (4) a region in which a polymer responsive to a temperature different from that in the region A is immobilized.

Item 13. The method of manufacturing a cell culture substrate according to item 12, wherein the region A and the region B on the surface of the substrate form a pattern.

Item 14. The method of manufacturing a cell culture substrate according to item 13, wherein the pattern comprises a line and space.

Item 15. The method of manufacturing a cell culture substrate according to any one of items 8 to 14, wherein a solvent for immobilizing the photopolymerization initiator on the surface of the cell culture substrate is concentrated sulfuric acid.

Item 16. A method of co-culturing two or more types of cells, the method comprising: using the cell culture substrate according to any one of items 1 to 7.

Item 17. A co-cultured cell sheet comprising two or more types of cells obtained by the co-culturing method according to item 16.

The cell culture substrate of the present invention has a surface which is hydrophilic and negatively charged because a sulfate group is introduced into the surface, and thus is considered to have excellent cell adhesion properties.

According to the present invention, a linear polymer can be immobilized only on an irradiated region when a region on a substrate surface irradiated with light is changed, and different polymers or different amounts of a polymer can be immobilized in a certain pattern on the substrate surface. Further, according to the present invention, for example, a complex pattern or shape pre-registered in a personal computer (hereinafter may be referred as to PC) may be projected with a projector so that the complex pattern or shape of different polymers or different amounts of a polymer can be easily formed on a substrate surface. Very complicated procedures required for functionalizing a surface are described above as a problem of the conventional method. The surface according to the present invention on which a photopolymerization initiator is immobilized may be manufactured using a ready-made polystyrene culture substrate and the like in fewer reaction steps. Therefore, the cost of industrial production may also be reduced. Further, photopolymerization can be completely performed in an aqueous system, and thus may be applicable to a cell culture substrate, which has been impossible in the conventional method. Furthermore, various cultured cells may be cultured with a maintained two-dimensional pattern, recovered and layered by using a cell culture substrate in which a linear polymer is immobilized on the surface according to the present invention. This may be applicable to the fields of biology, medicine, and regeneration medicine.

### BRIEF DESCRIPTION OF DRAWINGS

Fig. 1 shows measurement results from ultraviolet-visible absorption spectrometric analysis of a photopolymerization initiator-immobilized surface obtained in Example 1.
Fig. 2 shows results from X-ray photoelectron spectroscopic measurements of a polystyrene culture dish (Pst), a photopolymerization initiator-immobilized surface (TX-Pst) and a PIPAAm immobilized surface (1, 3, 5 wt% PIPAAm-Pst) obtained in Example 1.
Fig. 3 shows results from FT-IR measurements of a polystyrene culture dish (Pst), a photopolymerization initiator-immobilized surface (TX-Pst) and a PIPAAm immobilized surface (1, 3, 5 wt% PIPAAm-Pst) obtained in Example 1.
Fig. 4 shows phase-contrast microscope photographs of bovine vascular endothelial cells at specified time obtained in Example 2 adhered on a polystyrene surface for cell culture (TCPS), a PIPAAm immobilized surface (1, 3, 5 wt% PIPAAm-Pst).
Fig. 5 shows phase-contrast microscope photographs of bovine vascular endothelial cells at specified time obtained in Example 2 adhered on a 5 wt% PIPAAm-Pst and detached in a sheet form by low-temperature treatment.
Fig. 6 shows micrographs of fluorescent labeled BSA and bovine vascular endothelial cells obtained in Example 3 each adsorbed and adhered on a PAAm patterned (stripe) surface. Top panels are fluorescence images, and bottom panels are optical images.
Fig. 7 shows micrographs of fluorescent labeled BSA and bovine vascular endothelial cells obtained in Example 3 each adsorbed and adhered on a PAAm patterned (square) surface. A left panel is a fluorescence image, and a right panel is an optical image.
Fig. 8 shows phase-contrast microscope photographs of bovine vascular endothelial cells at specified time obtained in Example 4 adhered on a PAAm/PIPAAm patterned surface.
Fig. 9 shows results from X-ray photoelectron spectroscopic measurements of a polycarbonate culture dish (PC), a photopolymerization initiator-immobilized surface (TX-PC) and a PIPAAm immobilized surface (PIPAAm-PC) obtained in Example 5.

### DESCRIPTION OF EMBODIMENTS

The present invention provides a cell culture substrate in which a photopolymerization initiator is immobilized on a surface of the cell culture substrate, and a linear polymer is immobilized on a part or the entirety of the surface via the initiator. A Norrish II type photopolymerization initiator, which is thioxanthone, is used as the photopolymerization initiator in view of a projector described below, the ease of adhesion and detachment of cells and the like. Specifically, examples of it include 2-chloro thioxanthone, 2-isopropyl thioxanthone and the like. There is no particular limitation for a method of immobilizing the photopolymerization initiator on a substrate surface, but for example, a method is preferred comprising: dissolving thiosalicylic acid in concentrated sulfuric acid which is used as a catalyst, spreading it on a substrate surface, and heating. This is preferred because thioxanthone can be efficiently immobilized on the substrate surface. There is no particular limitation for concentrated sulfuric acid used therefor as long as a concentration of sulfuric acid is 90% or more. Similarly, there is no particular limitation for a concentration of thiosalicylic acid in concentrated sulfuric acid. Further, there is no particular limitation for temperature conditions when heating, but 50°C or more is preferred, and 60°C or more is more preferred, and the most preferred temperature is 65°C or more. When a reaction temperature is lower than 50°C, the efficiency of introducing thioxanthone onto a substrate surface may be reduced. There is also no particular limitation for an upper limit, but it is usually 90°C or less. There is also no particular limitation for an immobilized amount of thioxanthone on a substrate surface obtained.

For a material of a cell culture substrate used in the present invention, not only materials such as polystyrene, polymethylmethacrylate, polycarbonate, polyethylene terephthalate and the like which are commonly used for cell culture but also any polymer compound on which a certain shape can generally be conferred may be used. Shapes thereof are not limited to a cell culture dish such as a Petri dish, and may be a plate, a fiber, and a (porous) particle. Further, a shape of a container (a flask and the like) commonly used for cell culture and the like may be used.

When a photopolymerization initiator immobilized on a substrate surface obtained in this way is irradiated with light in the presence of monomer, the monomer undergoes graft polymerization at the initiator and immobilized as a linear polymer on the substrate surface. For the light used to do so, light having an emission wavelength peak of 400 nm or a longer wavelength of more than 400nm is used. Even when a blue LED having an emission wavelength peak in a longer wavelength of more than 400 nm was used, immobilization as a linear polymer was possible. There is no particular limitation for a device for generating the light, and a mask which covers the light may be used in order to isolate a region to be irradiated with the light from a region not to be irradiated. However, a maskless projector which does not use a shield, for example, as described in Japanese Patent Laid-Open No. 2006-39010 is suitable for the present invention because when it is used, a region of a different polymer and a region of different amounts of a polymer on a substrate surface as described below may be pre-designed using a PC, and may be directly projected. There is no particular limitation for a procedure to do so, but first, a monomer as described below is applied to a substrate surface on which a photopolymerization initiator has been immobilized, and light is projected to perform immobilization at only a place where a polymer thereof is to be immobilized on the substrate surface. Then, unreacted monomer is washed out, and an operation of drying the substrate surface is repeated if desired, thereby immobilization on the substrate surface can be performed with types and amounts of polymers freely changed.

There is no particular limitation for a monomer species (that is, a polymer species in which the monomers are polymerized) used in the present invention, but in a case where a temperature responsive polymer having hydration force changed at 0 to 80°C is immobilized on a substrate surface, cultured cells may be detached without significant damage, and may be detached as a cell sheet depending on a culture condition, as described above. Such temperature responsive polymers include a polymer having a minimum critical solution temperature (LCST) and a polymer having a maximum critical solution temperature (UCST), and may be any of a homopolymer thereof, a copolymer thereof, or a mixture thereof. Such polymers include, for example, a polymer described in Japanse Patent Laid-Open No. H06-104061. Specifically, for example, they can be obtained by homopolymerization or copolymerization of the following monomers. Monomers which can be used include, for example, (meth) acrylamide compounds, *N*- (or *N,N*-di) alkyl substituted (meth) acrylamide derivatives or vinyl ether derivatives, and polyvinyl alcohol partial acetylated materials, and for a copolymer, any two or more of these may be used. Further, copolymerization of monomers other than those described above, grafting or copolymerization of polymers each other, or a mixture of a polymer and a copolymer may be used. Cross-linking is also possible as long as the original properties of a polymer are not impaired. To this end, separation is performed in a range of 5°C to 50°C since a material to be separated is a biological material. Therefore, temperature responsive polymers include the followings: poly-*N-n*-propylacrylamide (a minimum critical solution temperature is 21°C for a homopolymer), poly-*N-n*-propylmethacrylamide (27°C, the same as above), poly-*N*-isopropylacrylamide (32°C, the same as above), poly-*N-*isopropylmethacrylamide (43°C, the same as above), Poly-*N*-cyclopropylacrylamide (45°C, the same as above), poly-*N-*ethoxyethylacrylamide (approximately 35°C, the same as above), poly-*N*-ethoxyethylmethacrylamide (approximately 45°C, the same as above), poly-*N*-tetrahydrofurfurylacrylamide (approximately 28°C, the same as above), poly-*N*-tetrahydrofurfurylmethacrylamide (approximately 35°C, the same as above), poly-*N,N-*ethylmethylacrylamide (approximately 56°C, the same as above), poly-*N,N-*diethylacrylamide (approximately 32°C, the same as above) and the like. An immobilized amount of a temperature responsive polymer in the present invention is preferably in a range of 0.8 to 3.0 µg/cm², more preferably 0.9 to 2.0 µg/cm², even more preferably 1.3 to 1.8 µg/cm². In a case where an immobilized amount is less than 0.8 µg/cm², cultured cells on the polymer may be difficult to be detached even when changing temperature, resulting in significantly decreased working efficiency. In contrast, in the case of more than 3.0 µg/cm², cells may be difficult to adhere to that region, and sufficient cell adhesion may be difficult to be achieved. An immobilized amount may be measured according to the conventional method. For example, the FT-IR-ATR method, the elemental analysis method, ESCA and the like may be used. Any method may be used.

According to the present invention, a temperature responsive polymer described above may be immobilized on the entire substrate surface, or may be immobilized on a part of the substrate surface. In a case where a temperature responsive polymer is immobilized to a part of a substrate surface, various cell culture conditions can be created by preparing a substrate surface having two regions: a region A in which the temperature responsive polymer is immobilized, and a region B comprising any one of (1) to (4) or any combination of two or three of (1) to (4):
(1) a region in which a polymer having higher affinity with cells than that in the region A is immobilized,
(2) a region in which a polymer having lower affinity with cells than that in the region A is immobilized,
(3) a region in which the temperature responsive polymer is immobilized in an amount different from that in the region A,
(4) a region in which a polymer responsive to a temperature different from that in the region A is immobilized.

Examples of a form in each region as observed from the above include, but not limited to, for example, (I) a line and space pattern, (II) a dot-like pattern, (III) a lattice-like pattern, other specifically shaped patterns, or mixed patterns thereof. Further, there is no particular limitation for a size of each region, but the longest distance (center-to-center distance) between cells present in adjacent regions A and B is preferably 1 cm or less, more preferably 0.05 mm to 8 mm, even more preferably 0.1 mm to 3 mm in a case where two or more different cells are co-cultured as described below. In the case of 1 cm or more, interactions between xenogeneic co-cultured cells may be weak, resulting in decreased co-culturing efficiency. In contrast, in the case of 0.05 mm or less, there may not be sufficient space for cells to grow in one of the regions A and B, or both, resulting in decreased co-culturing efficiency.

Examples of a polymer having high affinity with cells described in (1) above include, but not limited to, for example, those having an ionic group, a hydrophilic/hydrophobic group and the like; those having an ionic group, a hydrophilic/hydrophobic group and the like subjected to surface treatment such as glow discharge and corona discharge after immobilized on a substrate surface; as well as polymers of any or a combination of cell-adhesive proteins such as fibronectin, collagen, laminin and the like, or treated materials thereof.

There is no particular limitation for a cell-nonadhesive polymer having low affinity with cells described in (2) above as long as cells do not adhere to it, and examples of it include, for example, a hydrophilic polymer such as poly-*N*-acryloylmorpholine, polyacrylamide, polydimethylacrylamide, polyethylene glycol and cellulose, or a strongly hydrophobic polymer such as a silicone polymer and a fluorine polymer.

When a temperature responsive polymer adheres to both the regions A and B described in (3) above, the difference in the immobilized amounts between A and B is preferably at least 0.1 µg/cm² or more, more preferably 0.20 µg/cm² or more, and even more preferably 0.50 µg/cm² or more. In the case of 0.10 µg/cm² or less, when trying to detach cells in one region, cells in the other region may also be dragged and detached together. Meanwhile, a response is quicker even at the same temperature in a region where a greater amount of a polymer is immobilized. Therefore, if a treatment time is different, cells in only one region may be detached. According to the present invention, an immobilized amount may be larger in either the region A or B, and a support surface may be freely designed depending on purposes.

In a case where different temperature responsive polymers are immobilized in both the regions A and B as described in (4) above, the difference in temperatures at which a respective polymer in A and B responds needs to be at least 2°C or more, preferably 4°C or more, and even more preferably 8°C or more. In the case of 2°C or less, even when trying to detach cells on one region, cells in the other region may also be dragged and detached. Meanwhile, a response is quicker even at the same temperature in a region where a greater amount of a polymer is immobilized. Therefore, if a treatment time is different, cells in only one region may be detached. According to the present invention, an immobilized amount may be larger in either the region A or B, and a support surface may be freely designed depending on purposes.

According to the present invention, xenogeneic cells can also be co-cultured by culturing them on a patterned substrate. This may be achieved, for example, by a method comprising: immobilizing a polymer having high affinity with cells on a support surface as the region B, immobilizing poly-*N*-isopropylacrylamide (hereinafter referred to as PIPAAm) as the region A therein to design a temperature responsive region on/from which cells are adhered/detached. That is, it is an approach in which first, predetermined cells are cultured into a monolayer (a sheet) at 37°C, and then cells only in a temperature responsive domain are detached by decreasing temperature (for example, 25°C or less), and then different cells are inoculated after increasing temperature to 37°C again to create a xenogeneic cell region in stable predetermined cells which have already formed a domain. For example, in a mono-culture system of hepatic parenchymal cells, they start to gradually die after one week. In contrast, according to a method in which the cell culture support of the present invention is used, hepatic parenchymal cells and an endothelial cell line may be co-cultured over a period of two weeks or longer.

Cells used on a cell culture substrate surface obtained according to the present invention are preferably animal cells. There is no particular limitation for where they are available and how they are produced. Cells according to the present invention include, for example, animal cells, insect cells, plant cells and the like; and bacteria. In particular, origins of animal cells include, but not limited to, human, monkey, canine, feline, rabbit, rat, nude mouse, mouse, guinea pig, swine, sheep, Chinese hamster, bovine, marmoset, African green monkey and the like. Further, there is no particular limitation for culture medium used in the present invention as long as it is culture medium used for culturing animal cells, but examples of it include, for example, serum free culture medium, serum containing culture medium and the like. A differentiation-inducing agent such as retinoic acid and ascorbic acid may be further added to such culture medium. There is no particular limitation for an inoculation density on a substrate surface as long as it follows the conventional method.

### EXAMPLES

Below, the present invention will be described in more detail based on Examples. However, the present invention is not limited to these in any way.

### Example 1

### (Manufacture of temperature responsive polymer-immobilized surface using photopolymerization initiator)

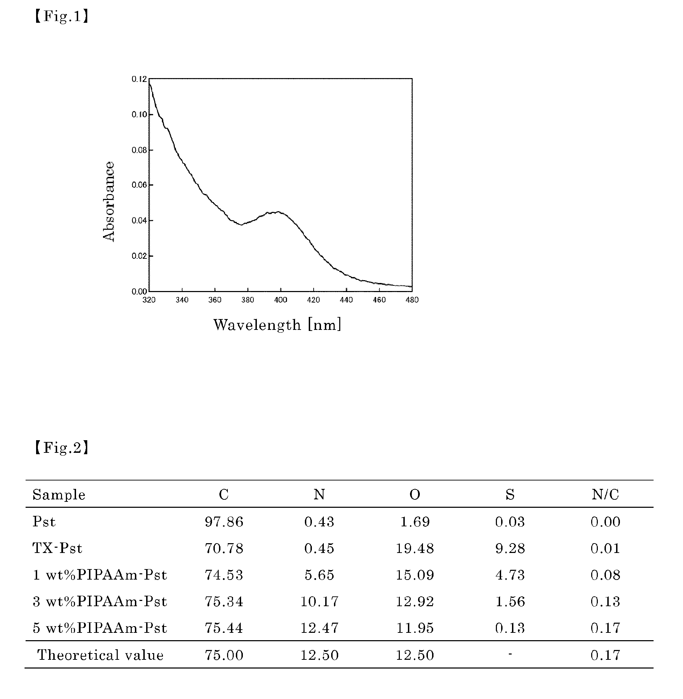

Thiosalicylic acid (Tokyo Chemical Industry Co., Ltd.) was slowly added to sulfuric acid (Wako Pure Chemical Industries, Ltd.) having a concentration of 95% to give a concentration of 20 mM, and stirred for 10 minutes to prepare a reaction solution. Then, a 2 mL reaction solution was added to each commercially available polystyrene Petri dish (Corning, Inc.), and left to stand for 1 hour at room temperature, and then heated at 65°C for 3 hours. The sample after the reaction was left to stand at room temperature over night to lower temperature. Then, an unreacted solution was removed and washed with pure water, and drying was performed in an oven at 45°C. An ultraviolet-visible absorption spectrum of a thioxanthone-immobilized polystyrene surface manufactured according to the present approach was measured (Fig. 1). (UV-3101 PC, Shimazu Corporation). Then, to a photopolymerization initiator-introduced polystyrene surface having a 0.5 mm-thick silicon rubber sheet placed thereon, dropwise added was an aqueous *N*-isopropylacrylamide (IPAAm) monomer solution to which 100 mM *N*-methyldiethanolamine had been added. A 23-mm squared cover glass was placed thereon to sandwich the monomer solution, and polymerized by irradiation with light having an emission wavelength peak at about 400 nm (light having a wavelength shorter than 400nm is cut with a filter) from a distance of about 10 cm. The light is visible light, but ultraviolet light having a wavelength shorter than 400 nm may be included depending on the performance of a filter and the like. A polymerization reaction was performed with a fixed irradiation time of 30 minutes and with varied concentrations of a monomer solution of 1, 3 and 5 wt%. As a light source, a 500 W extra high pressure mercury lamp was used with an energy intensity at a wavelength of 405 nm adjusted to 70 mW/cm². Further, a temperature adjustable stage was installed in a sample compartment, and the temperature was set to 15°C. After the reaction, the cover glass and the silicon rubber sheet were removed, and washed with pure water to remove unreacted monomers and unimmobilized polymers. After washing, drying was performed in an oven at 45°C to obtain a PIPAAm immobilized polystyrene surface. The resulting PIPAAm-Pst surface was used for analysis of a chemical composition with a X ray photoelectron spectrometer (XPS; K-alpha, ThermoFisher Scientific) and a Fourier transform infrared spectroscopy instrument (FT-IR; Spectrum One, Perkin Elmer).

### (Manufacture of PIPAAm immobilized surface and results from surface analysis)

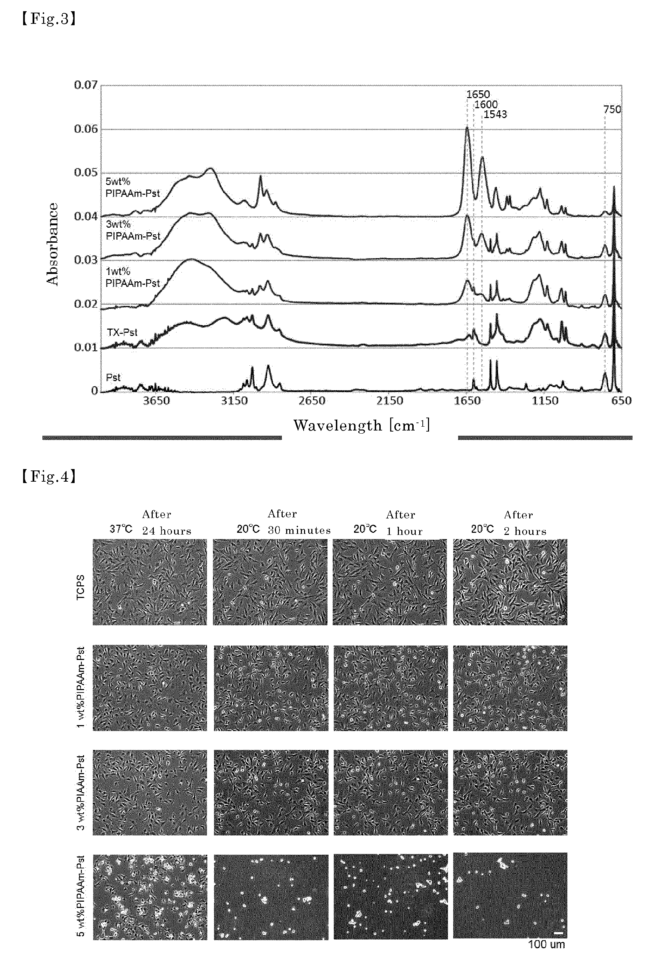

Fig. 1 shows an ultraviolet-visible absorption spectrum of a photopolymerization initiator-immobilized surface. The resulting surface was found to have a broad absorption region around 400 nm as compared with a Petri dish. Then, surface element composition ratios of a surface of a commercially available polystyrene culture dish (Pst), a photopolymerization initiator immobilized polystyrene surface (TX-Pst), and a PIPAAm immobilized polystyrene surface (1, 3, 5 wt% PIPAAm-Pst) were measured with an X ray photoelectron spectrometer (Fig. 2). The significantly increased ratio of S on the TX-Pst surface as compared with the Pst surface showed that the sulfuric acid group and the photopolymerization initiator (thioxanthone) were immobilized on the polystyrene surface. Further, since the element composition ratio of the PIPAAm-photopolymerized surface showed a decreased ratio of S and an increased ratio of N, and the value of N/C was also precisely consist to a theoretical value, PIPAAm was found to be immobilized on the polystyrene surface. Next, results from Fourier transform infrared spectroscopic measurements are shown in Fig. 3. A broad peak from thioxanthone was observed around 1150 cm⁻¹ in an IR spectrum for TX-Pst. Further, for the PIPAAm immobilized surface, peaks from amide in PIPAAm at 1650 and 1543 cm⁻¹ (Amide I and Amide II) were observed. Moreover, it was found that the amide peaks tended to increase as a concentration of the mother monomer increased. This revealed that an immobilized amount of a temperature responsive polymer may be controlled by changing a monomer concentration.

### Example 2

### (Cell culture on temperature responsive polystyrene surface produced by photopolymerization)

A temperature responsive surface produced as in Example 1 was sterilized with 70% ethanol or ultraviolet irradiation. Endothelial cells from bovine carotid artery (EC) were inoculated at a density of 5.0 x 10⁴ cells/cm², and cultured at 37°C for 24 hours using 10% FBS containing DMEM. Then, medium exchange was performed, and cell culture was performed for 2 hours in an incubator set at 20°C, and detachment behavior of the cells was observed under a phase-contrast microscope.

### (Results from cell culture on temperature responsive polystyrene surface produced by photopolymerization)

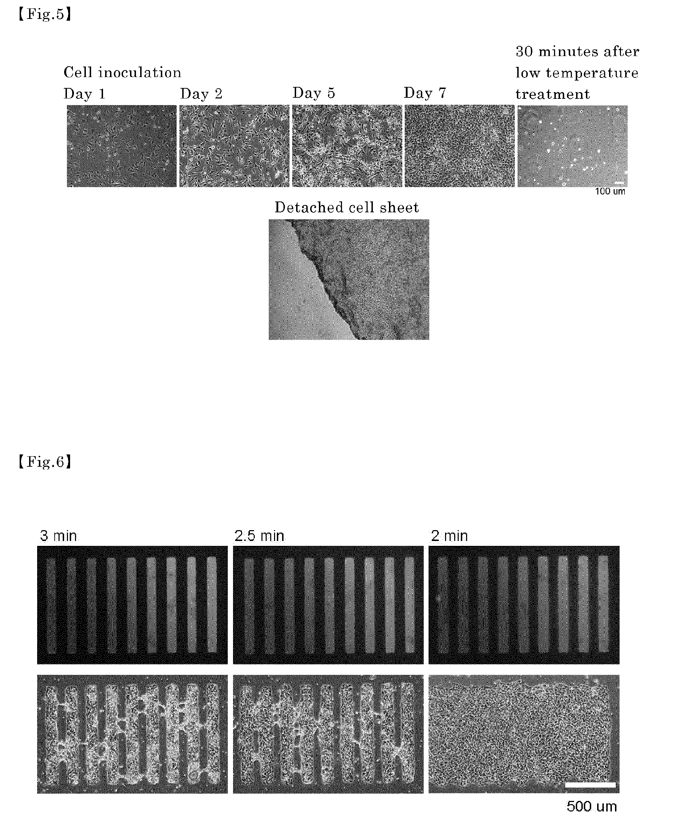

Cell adhesion was observed on a 1 wt% PIPAAm-Pst surface and a 3 wt% PIPAAm-Pst surface, but cell detachment when low temperature treatment was performed at 20°C was not observed even at 2 hours after the treatment. On the other hand, complete cell detachment was observed for a 5 wt% PIPAAm-Pst surface at about 30 minutes after the low temperature treatment (Fig. 4). Further, when cells were inoculated on a 5 wt% PIPAAm-Pst surface in a similar concentration, and cultured for 7 days, it was found that the cells grew to confluence and were detachable as a sheet-like form by the low temperature treatment (Fig. 5).

### Example 3

### (Preparation of hydrophilic patterned surface by photopolymerization using maskless irradiation device)

A 50 wt% aqueous acrylamide (AAm) monomer solution was sandwiched on a photopolymerization initiator-immobilized surface as in Example 2, and irradiated with visible light in a patterned way using a maskless irradiation device to polymerize a hydrophilic polymer AAm. After the reaction, the cover glass and the silicon rubber sheet were removed, and washed with pure water to remove unreacted monomers and unimmobilized polymers. After washing, drying was performed in an oven at 45°C to obtain a PAAm patterned surface. Next, a 100 µg/ml fluorescent labeled protein solution (Alexa 488-labeled bovine serum albumin; Alexa488-BSA) was adsorbed at room temperature, and the contrast in amounts of the protein adsorbed on the patterned surface was evaluated with fluorescence microscope images. Further, endothelial cells from bovine carotid artery (EC) were inoculated in a density of 5.0 x 10⁴ cells/cm², and cultured at 37°C for 24 hours using 10% FBS containing DMEM, and then adhesion behavior of the endothelial cells on a patterned PAAm surface was observed under a phase-contrast microscope.

### (Results from preparation of hydrophilic patterned surface by photopolymerization using maskless irradiation device)

It was found that a PAAm patterned surface (100 µm stripes) was able to be prepared by irradiation of visible light for 2.5 to 3 minutes, and fluorescent labeled

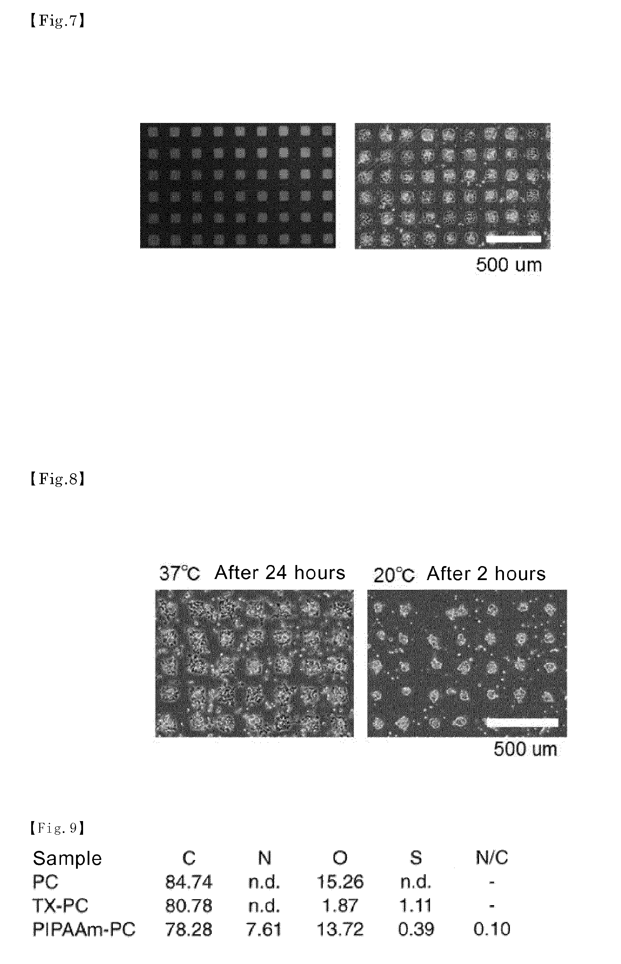

BSA and endothelial cells were selectively adsorbed and adhered outside the PAAm immobilized region (Fig. 6). Further, as shown in Fig. 7, it was found that a similar patterned surface was able to be prepared even when a form of patterns was changed to 100 µm squares and the like.

### Example 4

### (Preparation of temperature responsive/hydrophilic patterned surface by photopolymerization using maskless irradiation device)

A PAAm patterned surface of 100 µm squares was prepared as in Example 3. Next, a 20 wt% aqueous IPAAm monomer solution was sandwiched by a similar method, and irradiated with visible light in a pattern opposite to the first time using a maskless irradiation device to polymerize a temperature responsive polymer PIPAAm. Endothelial cells from bovine carotid artery (EC) were inoculated on the resulting temperature responsive/hydrophilic patterned surface in a density of 5.0 x 10⁴ cells/cm², and cultured at 37°C for 24 hours using 10% FBS containing DMEM. Then, medium exchange was performed, and cell culture was performed for 2 hours in an incubator set at 20°C, and detachment behavior of the cells was observed under a phase-contrast microscope.

### (Results from preparation of hydrophilic patterned surface by photopolymerization using maskless irradiation device)

As shown in Fig. 8, adhesion regions of endothelial cells were controlled by AAm in a patterned fashion on the PIPAAm/PAAm patterned surface. Further, it was found that the cells adhered on the PIPAAm regions were allowed to be detached by performing the low temperature treatment. The results suggest that a wide variety of polymers can be immobilized in a stepwise fashion by using the surface according to the present invention.

### Example 5

### (Introduction of photopolymerization initiator on polycarbonate surface)

A thioxanthone immobilized polycarbonate surface was prepared as in Example 1 except that a polycarbonate culture dish was used instead of a polystyrene Petri dish, and *N*-isopropylacrylamide (IPAAm) monomers were further polymerized on this surface to obtain a PIPAAm immobilized polycarbonate surface.

Element composition ratios of the polycarbonate culture dish surface (PC), the photopolymerization initiator immobilized polycarbonate surface (TX-PC) and the PIPAAm immobilized polycarbonate surface (PIPAAm-PC) were measured using an X ray photoelectron spectrometer (Fig. 9). As a result, the significantly increased ratio of S on the TX-PC surface as compared with the PC surface showed that the sulfuric acid group and the photopolymerization initiator (thioxanthone) were immobilized on the polycarbonate surface. Further, since the element composition ratio of the PIPAAm-photopolymerized surface showed a decreased ratio of S and an increased ratio of N, and the value of N/C was also precisely consist to a theoretical value, PIPAAm was found to be immobilized on the polycarbonate surface.

### Industrial applicability

According to the present invention, by changing a place to be irradiated with light on a substrate surface, a linear polymer can be immobilized only at an irradiated place, and different polymers and different amounts of a polymer can be easily immobilized in a patterned fashion on a substrate surface less expensively than the conventional method. Further, according to the present invention, for example, a complex pattern or shape pre-registered in a personal computer (hereinafter may be referred to as PC) may be projected with a projector so that the complex pattern or shape can be easily formed on a substrate surface.

## Claims

1. A cell culture substrate, comprising a photopolymerization initiator immobilized on a surface of the cell culture substrate, and a linear polymer immobilized on a part or the entirety of the surface via the photopolymerization initiator, and wherein the photopolymerization initiator is thioxanthone.

2. The cell culture substrate according to claim 1, wherein the linear polymer immobilized on the surface of the substrate comprises at least one temperature responsive polymer having hydration force changing between 0 and 80°C.

3. The cell culture substrate according to any one of claims 1 to 2, wherein said at least one temperature responsive polymer is poly(*N*-isopropylacrylamide).

4. The cell culture substrate according to any one of claims 1 to 3, having a surface comprising two regions:
a region A in which the temperature responsive polymer is immobilized, and
a region B comprising any one of (1) to (4) or any combination of two or three of (1) to (4):
(1) a region in which a polymer having higher affinity to cells than that in the region A is immobilized,
(2) a region in which a polymer having lower affinity to cells than that in the region A is immobilized,
(3) a region in which the temperature responsive polymer is immobilized in an amount different from that in the region A,
(4) a region in which a polymer responsive to a temperature different from that in the region A is immobilized.

5. The cell culture substrate according to claim 4, wherein the immobilized amount of the temperature responsive polymer on the surface of the region A is 0.8 to 10.0 µg/cm².

6. The cell culture substrate according to any one of claims 4 and 5, wherein the region A and the region B on the surface of the substrate form a pattern.

7. The cell culture substrate according to claim 6, wherein the pattern comprises a line and space.

8. A method of manufacturing a cell culture substrate, the method comprising:
immobilizing a photopolymerization initiator on a surface of the cell culture substrate,
irradiating the photopolymerization initiator-immobilized surface of the substrate with light having an emission wavelength peak of 400 nm or longer than 400 nm to immobilize a linear polymer on a part or the entirety of the surface at the initiator, wherein the photopolymerization initiator is thioxanthone.

9. The method of manufacturing a cell culture substrate according to claim 8, wherein different kinds of linear polymers and/or different amounts of linear polymers are immobilized on the surface of the substrate by changing light irradiation regions on the surface of the cell culture substrate without placing a shield on the surface of the cell culture substrate.

10. The method of manufacturing a cell culture substrate according to any one of claims 8 to 9, wherein the linear polymer immobilized on the surface of the substrate comprises at least one temperature responsive polymer having hydration force changing between 0 and 80°C.

11. The method of manufacturing a cell culture substrate according to claim 10, wherein said at least one temperature responsive polymers is poly(*N*-isopropylacrylamide).

12. The method of manufacturing the cell culture substrate according to any one of claims 8 to 11, wherein said cell culture substrate has a surface comprising two regions:
a region A in which the temperature responsive polymer is immobilized, and
a region B comprising any one of (1) to (4) or any combination of two or three of (1) to (4):
(1) a region in which a polymer having higher affinity to cells than that in the region A is immobilized,
(2) a region in which a polymer having lower affinity to cells than that in the region A is immobilized,
(3) a region in which the temperature responsive polymer is immobilized in an amount different from that in the region A,
(4) a region in which a polymer responsive to a temperature different from that in the region A is immobilized.

13. The method of manufacturing a cell culture substrate according to claim 12, wherein the region A and the region B on the surface of the substrate form a pattern.

14. The method of manufacturing a cell culture substrate according to claim 13, wherein the pattern comprises a line and space.

15. The method of manufacturing a cell culture substrate according to any one of claims 8 to 14, wherein a solvent for immobilizing the photopolymerization initiator on the surface of the cell culture substrate is concentrated sulfuric acid.

16. A method of co-culturing two or more types of cells, the method comprising: using the cell culture substrate according to any one of claims 1 to 7.

17. A co-cultured cell sheet comprising two or more types of cells obtained by the co-culturing method according to claim 16.
